# EUROPEAN PATENT SPECIFICATION

(11) **EP 2 262 495 B1**
(45) Date of publication and mention of the grant of the patent: **31.10.2018**
(21) Application number: 09715570.9
(22) Date of filing: 27.02.2009
(51) Int. Cl.: A61K 31/4015, A61K 31/205, A61K 31/122, A61P 25/28, A61P 25/16

(54) **PHARMACEUTICAL COMPOSITION COMPRISING RACETAM AND CARNITINE AND PROCESS FOR ITS PREPARATION**
PHARMAZEUTISCHE ZUSAMMENSETZUNG MIT RACETAM UND CARNITIN UND HERSTELLUNGSVERFAHREN DAFÜR
COMPOSITION PHARMACEUTIQUE COMPRENANT DU RACETAM ET DE LA CARNITINE ET SON PROCEDE DE PREPARATION

(30) Priority: 29.02.2008 BR PI0800432
(43) Date of publication of application: 22.12.2010
(73) Proprietor: Biolab Sanus Farmacéutica Ltda, 04071-900 Taboä da Serra - SP (BR)
(72) Inventor: PERREIRA, José Roberto da Costa, 04509-021 São Paulo-SP (BR); FALCI Marcio, 05641-900 São Paulo - SP (BR); ALARIO, Mauricio Matteis, 01405-000 São Paulo - SP (BR)
(74) Representative: Herzog, Fiesser & Partner Patentanwälte PartG mbB
(86) International application number: PCT/BR2009/000053
(87) International publication number: WO 2009/105853

(56) References cited:
- WO-A1-00/74675
- WO-A1-2006/048294
- WO-A2-2004/094375
- WO-A2-2006/110588
- US-A- 5 977 162
- MARCOFF ET AL: "The Role of Coenzyme Q10 in Statin-Associated Myopathy", JOURNAL OF THE AMERICAN COLLEGE OF CARDIOLOGY, ELSEVIER, NEW YORK, NY, US, vol. 49, no. 23, 5 June 2007 (2007-06-05), pages 2231-2237, XP022105696, ISSN: 0735-1097
- Santos: "IAD: diverse aspects of mitochondrial malfunctioning", , 25 June 2010 (2010-06-25), XP055335745, Retrieved from the Internet: URL:https://www.ncbi.nlm.nih.gov/pmc/artic les/PMC2907118/pdf/ijcep0003-0570.pdf [retrieved on 2017-01-16]
- Uta Keil ET AL: "Piracetam improves mitochondrial dysfunction following oxidative stress", BRITISH JOURNAL OF PHARMACOLOGY, vol. 147, no. 2, 1 January 2006 (2006-01-01), pages 199-208, XP055335849, ISSN: 0007-1188, DOI: 10.1038/sj.bjp.0706459
- Anonymous: "John Libbey Eurotext - Epileptic Disorders - Piracetam and levetiracetam: close structural similarities but different pharmacological and clinical profiles", , 30 June 2000 (2000-06-30), XP055335851, Retrieved from the Internet: URL:http://www.jle.com/fr/revues/epd/e-doc s/piracetam_and_levetiracetam_close_struct ural_similarities_but_different_pharmacolo gical_and_clinical_profiles_110118/article .phtml?tab=texte [retrieved on 2017-01-17]
- John G Pastorino ET AL: "THE JOURNAL OF BIOLOGICAL CHEMISTRY 0 1993 by The American Society for Biochemistry and Molecular Biology Cyclosporin and Carnitine Prevent the Anoxic Death of Cultured Hepatocytes by Inhibiting the Mitochondrial Permeability Transition*", , 5 July 1993 (1993-07-05), pages 13791-13796, XP055335854, Retrieved from the Internet: URL:http://www.jbc.org/content/268/19/1379 1.full.pdf#page=1&view=FitH
- Zbigniew K. Binienda: "Neuroprotective Effects of l-Carnitine in Induced Mitochondrial Dysfunction", Annals of the New York Academy of Sciences, vol. 993, no. 1, 1 May 2003 (2003-05-01), pages 289-295, XP055335857, US ISSN: 0077-8923, DOI: 10.1111/j.1749-6632.2003.tb07536.x
- A. Virmani: "Effects of Metabolic Modifiers Such as Carnitines, Coenzyme Q10, and PUFAs against Different Forms of Neurotoxic Insults: Metabolic Inhibitors, MPTP, and Methamphetamine", Annals of the New York Academy of Sciences, vol. 1053, no. 1, 1 August 2005 (2005-08-01), pages 183-191, XP055335858, US ISSN: 0077-8923, DOI: 10.1196/annals.1344.016

## Description

### Field of the Invention

The present invention refers to a use of a combination of racetam and L-carnitine, as in claim 1.

### Backround of Invention

In recent years, researchers has indicated a large spectrum of diseases associated with variations in mitochondrial function, including hereditary diseases, and mitochondrial dysfunction associated with aging, and neurodegenerative diseases related with the aging process, for example, Alzheimer's disease and Parkinson's Disease.

More details about medical conditions related with mitochondrial dysfunctions are found in the work of Maruszak and collegues (Maruszak, A., Gauweda-Walerich, K., Soltyszewski, I. and Zekanowski, C. "Mitochondrial DNA in pathogenesis of Alzheimer's and Parkinson's diseases". Acta Neurobiol Exp 2006, 66:153-176).

Also included in the medicial conditions related with mitochondrial dysfunctions are mitochondrial myopathies, that is, a group of neuromuscular diseases caused by mitochondria damage. In spite of not having specific treatments for mitochondrial myopathies, it is recommended, to lessen the symptoms of these diseases, physical therapy and therapies with vitamins, for example, riboflavin, with coenzyme Q (CoQ10) and with carnitine (see "Mitochondrial Miopathies Information Page", available on the Web at http://www.ninds.nih.gov/disorders/mitochondrial_myopathy/m itochondrial_myopathy.htm, accessed on 18/01/08).

Although there are still no available effective treatments for mitochondrial disturbances, new approaches are being developed involving gene therapy and metabolic therapy. In the case of the latter, the use of creatine presented positive results in a clinical study with 81 individuals (see Zeviani, M. and Di Donato, S. "Mitochondrial disorders", Brain 2004 127(10):2153-2172; Doi:10.1093/brain/awh259). Zeviani and Di Donato also mention that, although coenzyme Q10 (CoQ10) did not have effective action against mitochondrial diseases; it improves symptoms of CoQ10 deficiency. In other words, Zeviani and Donato confirmed a positive effect from the administration of coenzyme Q10 in cases in which the deficiency of this coenzyme impacts negatively medical conditions associated with mitochondrial disease.

It is important to note possible mitochondria damage caused by hypocholesterolemia therapy based on statins, as it was demonstrated that the referred drug promotes the transition of mitochondrial permeability (see, Old, J.A., Okanobo, H., Degasperi, G.R., Matsumoto, M.Y., Alberici, L.C., Cosso, R.G., Oliveira, H.C.F. And Vercesi, A.F. "Statins induce calcium-dependent mitochondrial permeability transition". Toxicology 219 (2006) 124-132).

The mitochondrial permeability transition (MPT) is a non-selective permeabilization process of the innermost mitochondrial membrane, and is typically promoted by the excessive accumulation of calcium ions, and by several compounds and medical conditions. Permeabilization of the innermost mitochondrial membrane as in MPT results in loss of components of the mitochondrial matrix, damage of mitochondrial functionality, and substantial swelling of organelles; with consequent rupture of the most external mitochondrial membrane and liberation of cytochrome C.

The relation between the excessive accumulation of mitochondrial calcium and MPT is oxidative stress. The confirmation of this fact is based on a large number of experiments using intact cells in which cell death induced by conditions involving MPT can be avoided by the use of antioxidants (see A.J. Castilho R.F. And Vercesi A.E. Mitochondrial permeability transition and oxidative stress-Kowaltowski - FEBS Letters 495 (2001) 12-15).

MPT induced by calcium is increased by several compounds denominated inductors, which include inorganic phosphates, pyridinic nucleotide oxidants, statins, and thyroid hormones. Most of these inductors are able to increase mitochondrial oxidative stress induced by calcium or react with tiol groups of membrane proteins.

Molecules of the racetam group are defined as a class of nootropic drugs that possess a pyrrolidine nucleus. The term "nootropic drugs" refers to a class of compounds that increase cognitive performance in human beings. The referred molecules activate glutamate type receptors that are co-localized with cholinergic receptors, promoting thus its activity in improvement of human cognitive performance.

Piracetam (2-oxo-pyrrolidone) was the first drug of the racetam group identified in the middle of 1960s (see patent GB1039113). The referred compound is normally used in dementia caused by Alzheimer's and other neurodegenerative diseases and/or associated with aging and the reduction of cognitive capacities and of memory.

One of the most important characteristics of piracetam is its capacity to improve cerebral energy, especially in conditions of deficient ATP (adenosine triphosphate).

The results of experiments in rats realized by Bogolepov (Bogolepov NN, Gusev EI, Burd GS, Buklina SB. [Ultrastructural aspects of acute cerebral ischemia during nootropil administration (experimental study)] Zh Nevropatol Psikhiatr Im S S Korsakova. 1983;83(7):984-90) suggest the existence of a connection between the capacity of Piracetam to normalize the metabolism of ATP, to stimulate the synthesis of phospholipids and ribosome function, and to increase glucose utilization.

Conforming with the findings of Bogolepov, other studies realized in animals showed that Piracetam accelerates animal recovery from hypoxic states. A controlled clinical study, published in 1989, indicated "a significantly positive effect" of Piracetam to increase cerebral blood flow, cerebral oxygen usage metabolic rate, and cerebral glucose metabolic rate in chronic impaired human brain function, as in the case of senile dementia of the Alzheimer type (see South MA James, "Piracetam - the original nootropic", available on the Web at http://www.smart-drugs.com/ias-piracetam.htm, accessed on 18/01/08).

Also important are the findings of Novikov (Novikov VE, Kovaleva LA. [The effect of nootropic agents on brain mitochondrial function in dynamics of craniocerebral trauma from the acts aspect] Eksp Klin Farmakol. 1998 Mar-Apr;61(2):65-8) and Gabryel (Gabryel B, Adamek M, Pudelko A, Malecki A, Trzeciak HI. Piracetam and vinpocetine exert cytoprotective activity and prevent apoptosis of astrocytes in vitro in hypoxia and reoxygenation. NeuroToxicol (2002) 1:19-31). Novikov and collegues showed that Piracetam avoids mitochondrial modifications and improves the capacity of mitochondria during development of traumatic edema of the brain. Later, Gabryel and collegues, in a study *in vitro* about one possible protective action of Piracetam and Vinpocetina against damage from hypoxia for astrocytes, verified that these drugs significantly reduce the number of apopototic cells during conditions of hypoxia. The apopototic effect of these drugs was supported by the following observations: (i) stimulus of mitochondrial function, (ii) increase of intracellular ATP, and (iii) inhibition of caspase-3 activity.

With these beneficial characteristics, Piracetam has been suggested for use in different treatments of neural disturbances. For example, document US5668117 mentions that Piracetam can be combined with carbonyl capture agents for the treatment of neurological diseases and clinical symptomology etiologically related with such diseases where it can have possible action on the degeneration of neurons associated with the aging process. Additionally, document US7226916 describes Piracetam included in pharmaceutical preparations for the treatment of degeneration of the cerebral function, and these preparations can include coenzyme Q10.

Keil and collegues (see Keil U., Scherping I., Hauptmann S., Schuessel K., Eckert A., Müller W.E. "Piracetam improves mitochondrial dysfunction following oxidative stress". British Journal of Druglogy (2006) 147, 199-208. Doi:10.1038/sj.bjp.0706459; published online on November 14, 2005) realized an important work on Piracetam's benefits related to the actions of nootropics and the reduction of neuropathologic symptoms caused by aging. In this publication, it is mentioned that this drug has the capacity of improve mitochondria dysfunction associated with oxidative stress and/or with the aging.

For carnitine, also known as vitamin BT, is a compound of quaternary ammonium that is present, mostly, in muscle of vertebrate animals, being produced *in vivo.* Carnitine is involved in the process of transfer of fatty acids through mitochondrial membranes. Carnitine deficiency is a medical condition that prevents the body of using fat to produce energy. Carnitine can be utilized in the forms D-carnitine, L-carnitine, D,L-carnitine, acetyl-L-carnitine, alkanoyl-L-carnitine, propionyl-L-carnitine, butyryl-L-carnitine, valeryl-L-carnitine and isovaleryl-L-carnitine (see "Primary carnitine deficiency", available on Web at http://ghr.nlm.nih.gov/condition=primarycarnitinedeficienc; and "Carnitine Information Page", available on the Web at http://www.anyvitamins.com/carnitine-info.htm, accessed on 18/01/08; and WO 9901126). It is also known that acetyl-L-carnitine has action on neurodegenerative diseases, such as Alzheimer's and Parkinson's (see "Acetyl-L-carnitine", available at http://www.pdrhealth.com/drugs/altmed/altmed-mono.aspx?contentFileName=ame0463.xml&contentName=Acetyl-L-carnitine&contentId=618, accessed on 18/01/08).

Pastorino and collegues (see Pastorino JG, Snyder JW, Serroni A, Hoek JB, Farber JL. Cyclosporin and carnitine prevent anoxic death of cultured hepatocytes by inhibiting mitochondrial permeability transition. J Biol Chem. 1993 Jul 5;268(19):13791-8) studied the effect of L-carnitine on mitochondrial damage. They showed, in their study, that there is a narrow correlation between transport inhibition of mitochondrial electons resulting from treatment with L-carnitine and the prevention of permeability transition in mitochondria isolated of cultured hepatocytes. The capacity of L-carnitine to protect hepatocytes in culture is a consequence of its capacity to avoid mitochondrial permeability transition. Therefore, cell death induced by anoxia as much as the inhibition of electron transport are associated with mitochondrial modifications related with an independent mechanism of maintenance of membrane potential and of cellular reserves of ATP.

Coenzyme Q, also denominated of co-enzyme Q10 (2,3,dimethoxyl-5-methyl-6-decaprenil-1,4-benzoquinone), CoQ10, ubiquinone, ubidecarenone, and vitamin Q10, is a compound derived from benzoquinones encountered in the majority of the human tissues and that has an important role in the productin of ATP. It is well-known that CoQ10 has action, proved scientifically, on several medical conditions, such as hypertension, Alzheimer's disease, angina, cardiomyopathy, muscular dystrophy, Parkinson's disease, among anothers (see: "Introduction have Coenzyme Q10" at http://faculty.washington.edu/ely/coenzq10.html, and "Coenzyme Q10", available on the Web at http://www.mayoclinic.com/health/coenzyme-q10/NS_patient-coenzymeq10, both accessed on 18/01/08).

Because of their beneficial actions on metabolism, carnitine and coenzyme Q10 have been used as therapies for medical conditions that involve neurodegenerative and cardiopathic processes, especially when the individual under treatment needs to use a drug of the statins group.

The prescription of statins is frequently aimed at the effective prevention and reduction of the risk of development of coronary artery diseases by the reduction of blood levels of chloresterol. Despite the quantity of clinical tests that revealed effectiveness and relative safety of statins, several adverse effects have been reported: the most concerning relate to hepatic function, to skeletal muscle, and to peripherial nerves. Myopathies are the adverse effects related with the most frequency. In fact, the principle reason for discontinuation of hypocholesterolemia treatment with statins is, in general, the appearance of muscular pain.

It is known that statins cause adverse effects, especially in the regime of prolonged treatment. Chapman and Carrie (Chapman, J. and Carrie, A.; "Mechanisms of Statin-Induced Myopathy - A Role for the Ubiquitin-Proteasome Pathway?"; Arterioscler. Thromb. Vasc. Biol. 2005; 25; 2441-2444) detailed a report about the side effects caused by statins, especially those related with cholesterol therapy and of the disturbances of the metabolism of ubiquinone (CoQ10) on human skeletal muscle and mitochondrial function.

In view of these adverse effects, it was proposed the combination of statins with drugs with action on the metabolism and/or mitochondrial function, essentially CoQ10 and carnitine. The following can be cited as examples of such associations:
- Coenzyme Q10 and statin - described in WO0243721; US2002058026; WO0185155; US5316765; EP383432; and Carnitine and statin - described in WO2000074675; WO9901126

Another example representative of the adverse effects caused by therapeutic agents associated with mitochondrial and/or metabolic disturbances is the group of antiepileptic drugs (anticonvulsants). Is known that these therapeutic agents influence negatively bone metabolism in view of several biochemical abnormalities (of bone metabolism), including hypocalcemia, hypophosfatemia, low levels of vitamin D, and an increase in PTH (hormone for the thyroid) (see, Valsamis, H.A., Arora, S.K., Labban, B. and McFarlane, S.I. "Antiepileptic Drugs and Metabolism". Nutrition & Metabolism 2006, 3:36).

US 5'977'162 A relates to a nutritional supplement for enhancing mitochondrial function in cells, which includes 10-1000 mg of alpha-lipoic acid, 10-1000 mg acetyl-L-carnitine, 15-360 mg coenzyme Q-10, and 15-360 mg glutathione.

As discussed in the Background of the Invention, it is not available, until now, a drug combination that joins the beneficial effects of compounds of the racetam class (for example, piracetam) with those of carnitine. Additionally, there is no medication that combines such association with CoQ10, or with therapeutic agents related to mitochondrial and/or metabolic disturbances, aiming at reducing the adverse effects of therapies based on agents for treatment of said disturbances, for example, hypocholesterolemic agents, hypoglycemic agents, antiepileptic agents, and others.

### Summary of the Invention

The present invention aims to provide a use as claimed in claim 1.

The invention provides a use of a pharmaceutical composition based on the synergistic effect of the combination of at least one compound of the racetam class with L-carnitine. Additionally, a preferred embodiment of the invention provides a combination of at least one compound of the racetam class and L-carnitine with coenzyme Q10, and optionally, a therapeutic agent associated with mitochondrial and/or metabolic disturbances to minimize the adverse effects of said agent. The combination of at least one compound of the class racetam + L-carnitine and CoQ10 and a therapeutic agent associated with mitochondrial and/or metabolic disturbances can be in the same pharmaceutical form or in separate pharmaceutical forms.

### Brief Description of the Figures

**Figure 1****.** Effect of different doses of Simvastatin on mitcochondrial swelling demonstrated through a dose response curve of calcium and simvastatin.
**Figure 2****.** Effect of the combination of L-carnitine and Piracetam on mitochrondrial swelling induced by Simvastatin.
**Figure 3****.** Effect of the combination of L-carnitine and Piracetam on mitochrondrial swelling induced by Pravastatin.
**Figure 4****.** Effect of the combination of L-carnitine and Piracetam on mitochrondrial swelling induced by Lovastatin.
**Figure 5****.** Effect of coenzyme Q on mitochrondrial swelling induced by Simvastatin.

### Detailed Description of the Invention

The present invention relates to the treatment of medical conditions involving mitochondrial disturbances, including mitochondrial myopathies, induced by a statin, a pharmaceutically accepatble salt thereof or an isomer thereof, in a mammal.

Some definitions of employed terms in the description and claims are supplied as follows:
- Therapeutic agent associated with mitochondrial and/or metabolic disturbance has the intention to mean a drug that is used in the therapy of minimizing or reversing a medical condition involving mitochrondrial or metabolic dysfunction, as, for example, myopathy, neuropathy, hypercholesterolemia, hyperglycemia, and epilepsy. Examples of therapeutic agents are: hypocholesterolemic agents, for example, statins; pyridinic nucleotide oxidants, for example, acetoacetate; thyroid hormones; and antiepileptic agents.
- Therapeutically effective quantity has the intention to mean the daily dose necessary for the minimization or reversion of a medical condition involving a mitochondrial and/or metabolic dysfunction.
- Pharmaceutically acceptable salts has the intention to mean the salt forms of the principle active ingredients of the composition of the invention, including, but not being limited to (i) non-toxic acid-addition salts formed by said active ingredients with inorganic acids, such as, hydrochloric acid, hydrobromic acid, sulfuric acid, and phosphoric acid, or with organo-carboxylic acids, such as gluconate, or with organo-sulphonic acids, such as mesylate and besylate, (ii) metal salts, preferably non-toxic besylate, (ii) metal salts, preferably non-toxic alkaline metal salts, including sodium or potassium salts, formed by said active ingredients with bases of these metals and pH adjusted to the appropriate value.
- Pharmaceutically acceptable vehicle has the intention to mean any inert substance or substances used as a vehicle or diluent for any one of the active ingredients of the composition of the present invention.
- RACETAM has the intention to mean nootropic drugs that possess a pyrrolidine nucleus. Such compounds include, but are not restricted to, Piracetam, Luracetam, Fasoracetam, Nebracetam Rolziracetam, Nefiracetam, Levetiracetam, Etiracetam Pramiracetam, Oxiracetam, Aniracetam, Phenylpiracetam, Coluracetam, Brivaracetam, Seletracetam, Rolipram, pharmaceutically acceptable salts thereof, and isomers thereof.
- Inhibitor of HMG CoA has the intention to mean any substance adequate for human and veterinarian use that promotes the blockade of enzyme 3-hydroxy-3-methyl-glutaryl-coenzyme A redutase, inhibiting, therefore, cholesterol. The said substances include, but are not limited to, statins, which include, but are not restrict to, simvastatin, lovastatin, atorvastatin, pravastatin, fluvastatin, dalvastatin, cerivastatin, mevastatin, pitavastatin, rosuvastatin, pharmaceutically acceptable salts thereof; and isomers thereof.

The use of a pharmaceutical composition as claimed is based on the unexpected synergism between at least one drug of the racetam group and L-carnitine, synergism that enables the treatment of mitochondrial disturbances concomitantly with the therapy of CoQ10 deficiency and of metabolic dysfunctions, such as for hypercholesterolemia, diabetes of metabolic syndrome, and neurodegenerative dysfunctions associated with the aging process.

To confirm the synergism of the combination of at least one active ingredient of the racetam group with L-carnitine, experiments were realized in vitro using small doses of piracetam, L-carnitine and piracetam + L-carnitine. Surprisingly the combination of these two drugs, applied at the same dose and administered separately, presented significant protective action for mitochondria, from damage induced by simvastatin. As mentioned previously, statins have, as an adverse effect, deleterious action on mitochondria, which is one of the causes of MPT, and myopathies that appear in medium- to long-term hypercholesterolemia treatment.

The concentration of piracetam, in the composition of the present invention, can correspond from a daily dose of 100mg to 12g, and can be in a single dose or multiple doses in appropriate pharmaceutical forms.

The concentration of L-carnitine can correspond to a daily dose from 100mg to 6g, and can be a single dose or distributed in multiple doses.

The concentration of coenzyme Q10 in the composition of the invention can correspond to a daily dose from 5mg to 200mg, in a single dose or multiple dose.

Additionally, according to the invention, the ratio of the components of the L-carnitine:racetam combination, for example, piracetam, can vary in the range from 1:1 to 1:10. Preferred ratios of L-carnitine:racetam, for example, piracetam, are 1:1 or 1:1,3 or 1:1:2 when present with CoQ10.

The agent of metabolic therapy as used according to the invention can be any drug directed to the treatment of dysfunction of metabolism, such as hypercholesterolemia. Preferentially, the said agent of metabolic therapy is selected from the group of statins that includes: simvastatin, lovastatin, atorvastatin, pravastatin, fluvastatin, dalvastatin, cerivastatin, mevastatin, pitavastatin, rosuvastatin, pharmaceutically acceptable salts thereof, and isomers thereof. The concentration of the agent of metabolic therapy can correspond to a daily dose usually used in antilipemic medications.

The combination of piracetam + L-carnitine, can include, additionally, a therapeutic agent related to mitochondrial and/or metabolic disturbance, with or without CoQ10) and a vehicle that besides pharmaceutically acceptable should be compatible with other formulation components, and said compositions can be in any pharmaceutical form.

Examples of such pharmaceutical forms are (i) tablets, optionally coated, chewable, effervescent, multicoated or soluble; (ii) pills; (iii) powder, optionally dispersable or effervescent; (iv) capsules of any kind, such as a hard gelatinous capsule, a soft gelatinous capsule, and an amylaceous capsule; (v) pastilles; (vi) granules, optionally in the form of microparticles or microcapsules, or in vectorized preparations, such as, lipossomos; (vii) suppositories, (viii) solutions, optionally oral, nasal, ophthalmic; (ix) injectable (including subcutaneous, intradermal, intermuscular and intravenous); (x) suspensions; (xi) syrups; (xii) infusion; and others. The solutions, suspensions, or syrups of oral administration can be, for example, in pharmaceutical presentations of vials, flasks, ampules, etc. Also included in the present invention are compositions of fast action, of prolonged action, and of delayed action. The preferred pharmaceutical forms of the invention are the solutions or suspensions for oral administration, preferentially in the pharmaceutical presentation of a vial, of glass or plastic.

In the case of pharmaceutical forms as a solution or suspension for oral administration, the pharmaceutically acceptable vehicle can include solvents and co-solvents, buffers, preservatives, coloring, flavorings, sweeteners, reducing agents, thickening agents, sequestering agents, surfactants, components for pH adjustment (for example, hydrochloric acid, sodium hydroxide), suspension agents, antioxidants, among others. The solvents or means of suspension can be: purified water and/or other hydrophilic solvents (for example, ethanol, DMSO, propylene glycol, PEG) or hydrophobic solvents (for example, oils). Co-solvents can be: ethanol, propylene glycol, glycerol, among others. Preservatives can be: phenols, parabens, and benzoic acid. The reducing agents can be: vitamin E, ascorbic acid, etc. The essential is that any of these excipients or additives be within the demands of quality for human and veterinarian use as established by competent authorities.

In the case of pharmaceutical forms as a tablet, they are able to include one or more excipients selected from the group consisting of diluents, disintegrants, agglutinants, coloring, and flavoring agents. The diluent can be one or more of calcium carbonate, calcium phosphate dibasic, calcium phosphate tribasic, calcium sulfate, microcristaline cellulose, pulverulent cellulose, dextrates, dextrins, dextrose excipients, frutose, kaolin, lactitol, lactose, manitol, sorbitol, starch, pregelatinized starch, saccharose, compressible sugar and confectioner's sugar, and in particular it can be lactose. The agglutinant can be one or more of methylcellulose, hydroxypropylcellulose, hydroxypropyl methylcellulose, polyvinylpirrolidone, gelatin, arabic gum, ethylcellulose, polyvinylic alcohol, pullulan, pregelatinized starch, agar, gum tragacanth, derivatives of alginic acid and propylene glycol, and alginate, and in particular it can be polyvinylpirrolidone. Disintegrants can be one or more of low-substituted hydroxypropyl cellulose, carboxymethyl cellulose, calcium carboxymethyl cellulose, sodium carboxymethyl cellulose, croscarmelose sodium, starch, sodium amidoglycolate, crystalline cellulose, hydroxypropyl starch, and partially pregelatinized starch.

The pharmaceutical compositions as used in the present invention can be prepared by processes known in the state of the art.

The term mitochondrial disturbance has the intention to mean all mitochondrial dysfunctions susceptible to systemic treatment, including mitochondrial myopathies.

Additionally, the use of the invention embraces therapies of metabolic dysfunctions that involve the use of drugs with adverse effects, which affect mitochondria and which generate or amplify cytopathologies, such as myopathies and neurodegenerative disturbances. An important example of the method of the invention is hypercholesterolemia therapy with the use of the composition of the invention in that the therapeutic agent associated with mitochondrial and/or metabolic disturbance is a statin of the group that includes, but is not limited to, simvastatin, lovastatin, atorvastatin, pravastatin, fluvastatin, dalvastatin, cerivastatin, mevastatin, pitavastatin, rosuvastatin, pharmaceutically acceptable salts thereof, and isomers thereof.

The present invention also relates to the administration of the composition of the invention in a single dose or multiple doses corresponding to a daily dose necessary to attenuate or eliminate the mitochondrial and/or metabolic dysfunction. This dosage can be in the range of 100mg to 12g for piracetam; in the range of 100mg to 6g for L-carnitine; and in the range of 5mg to 200mg for coenzyme Q10.

Additionally, the invention refers to a composition comprising: (a) the combination of at least one active ingredient of the racetam group, for example, piracetam or a pharmaceutically acceptable salt thereof or an isomer thereof with L-carnitine or a pharmaceutically acceptable thereof or an isomer thereof; and (b) substances that interfere in cellular metabolism. Examples of such substances are methyl radical donors, such as acetyl methionine, betaine and folic acid in doses normally used in pharmaceutical preparations, for example, 400mg (acetyl methionine), 500mg (betaine) and 800mg (folic acid) .

The invention also relates to a use of a pharmaceutical composition comprising:
(a) a therapeutically effective quantity of piracetam, a pharmaceutically acceptable salt thereof, or an isomer thereof;
(b) a therapeutically effective quantity of L-carnitine, a pharmaceutically acceptable salt thereof, or an isomer thereof; and
(c) a pharmaceutically acceptable vehicle as a second major embodiment.

In a further embodiment according to the second major embodiment, the pharmaceutical composition according to the second major embodiment further comprises a therapeutically effective quantity of coenzyme Q10.

In a further embodiment according to the second major embodiment, the pharmaceutical composition according to the second major embodiment further comprises a therapeutically effective quantity of one or more therapeutic agents associated with mitochondrial and/or metabolic disturbance.

In a further embodiment according to the second major embodiment, the ratio of L-carnitine with respect to piracetam ranges from 1:1 to 1:10.

In a further embodiment according to the second major embodiment, said therapeutic agent associated with a hypocholesterolemic agent.

In a further embodiment according to the second major embodiment, said statin is selected from the group consisting of simvastatin, lovastatin, atorvastatin, pravastatin, fluvastatin, dalvastatin, cerivastatin, mevastatin, pitavastatin, rosuvastatin, a pharmaceutically acceptable salt thereof, or an isomer thereof.

In a further embodiment according to the second major embodiment, said statin is simvastatin, a pharmaceutically acceptable salt thereof, or an isomer thereof.

In a further embodiment according to the second major embodiment, said statin is lovastatin, a pharmaceutically acceptable salt thereof, or an isomer thereof.

In a further embodiment according to the second major embodiment, said statin is pravastatin, a pharmaceutically acceptable salt thereof, or an isomer thereof.

Also preferred is a use of a pharmaceutical composition comprising:
(a) a therapeutically effective quantity of piracetam, a pharmaceutically acceptable salt thereof, or an isomer thereof;
(b) a therapeutically effective quantity of L-carnitine, a pharmaceutically acceptable salt thereof, or an isomer thereof;
(c) a therapeutically effective quantity of coenzyme Q10; and
(d) a pharmaceutically acceptable vehicle as a third major embodiment.

In a further embodiment according to the third major embodiment, the pharmaceutical composition further comprises a therapeutically effective quantity of one or more therapeutic agents associated with mitochondrial and/or metabolic disturbance.

In a further embodiment according to the third major embodiment, the ratio of L-carnitine with respect to piracetam ranges from 1:1 to 1:10.

In a further embodiment according to the third major embodiment, said therapeutic agent associated with a hypocholesterolemic agent.

In a further embodiment according to the third major embodiment, said therapeutic agent associated with mitochondrial and/or metabolic disturbance is a hypocholesterolemic agent.

In a further embodiment according to the third major embodiment, said hypocholesterolemic agent is a statin, a pharmaceutically acceptable salt thereof, or an isomer thereof.

In a further embodiment according to the third major embodiment, said statin is selected from the group consisting of simvastatin, lovastatin, atorvastatin, pravastatin, fluvastatin, dalvastatin, cerivastatin, mevastatin, pitavastatin, rosuvastatin, a pharmaceutically acceptable salt thereof, or an isomer thereof.

In a further embodiment according to the third major embodiment, said statin is simvastatin, a pharmaceutically acceptable salt thereof, or an isomer thereof.

In a further embodiment according to the third major embodiment, said statin is lovastatin, a pharmaceutically acceptable salt thereof, or an isomer thereof.

In a further embodiment according to the third major embodiment, said statin is pravastatin, a pharmaceutically acceptable salt thereof, or an isomer thereof.

Also preferred is a use of a pharmaceutical composition comprising:
(a) a therapeutically effective quantity of piracetam, a pharmaceutically acceptable salt thereof, or an isomer thereof;
(b) a therapeutically effective quantity of carnitine, a pharmaceutically acceptable salt thereof, or an isomer thereof;
(c) a therapeutically effective quantity of one or more therapeutic agents associated with mitochondrial and/or metabolic disturbance; and
(d) a pharmaceutically acceptable vehicle in a fourth major embodiment.

In a further embodiment according to the fourth major embodiment, the pharmaceutical composition further comprises a therapeutically effective quantity of coenzyme Q10.

In a further embodiment according to the fourth major embodiment, the ratio of carnitine with respect to piracetam ranges from 1:1 to 1:10.

In a further embodiment according to the fourth major embodiment, said therapeutic agent associated with a hypocholesterolemic agent, said hypocholesterolemie agent is a statin, a pharmaceutically acceptable salt thereof, or an isomer thereof.

In a further embodiment according to the fourth major embodiment, said statin is selected from the group consisting of simvastatin, lovastatin, atorvastatin, pravastatin, fluvastatin, dalvastatin, cerivastatin, mevastatin, pitavastatin, rosuvastatin, a pharmaceutically acceptable salt thereof, or an isomer thereof.

In a further embodiment according to the fourth major embodiment, said statin is simvastatin, a pharmaceutically acceptable salt thereof, or an isomer thereof.

In a further embodiment according to the fourth major embodiment, said statin is lovastatin, a pharmaceutly acceptable salt thereof, or an isomer thereof.

In a further embodiment according to the fourth major embodiment, said statin is pravastatin, a pharmaceutically acceptable salt thereof, or an isomer thereof.

Also preferred is a use of a pharmaceutical composition comprising:
(a) a therapeutically effective quantity of piracetam, a pharmaceutically acceptable salt thereof, or an isomer thereof;
(b) a therapeutically effective quantity of L-carnitine, a pharmaceutically acceptable salt thereof, or an isomer thereof;
(c) a therapeutically effective quantity of coenzyme Q10;
(d) a therapeutically effective quantity of at least one therapeutic agent associated with mitochondrial and/or metabolic disturbance; and
(e) a pharmaceutically acceptable vehicle as a fifth major embodiment.

In a further embodiment according to the fifth major embodiment, the ratio of carnitine with respect to piracetam ranges from 1:1 to 1:10.

In a further embodiment according to the fifth major embodiment, said therapeutic agent associated with a hypocholesterolemic agent, wherein said hypocholesterolemic agent is a statin, a pharmaceutically acceptable salt thereof, or an isomer thereof.

In a further embodiment according to the fifth major embodiment, said statin is selected from the group consisting of simvastatin, lovastatin, atorvastatin, pravastatin, fluvastatin, dalvastatin, cerivastatin, mevastatin, pitavastatin, rosuvastatin, a pharmaceutically acceptable salt thereof, or an isomer thereof.

In a further embodiment according to the fifth major embodiment, said statin is simvastatin, a pharmaceutically acceptable salt thereof, or an isomer thereof.

In a further embodiment according to the fifth major embodiment, said statin is lovastatin, a pharmaceutically acceptable salt thereof, or an isomer thereof.

In a further embodiment according to the fifth major embodiment, said statin is pravastatin, a pharmaceutically acceptable salt thereof, or an isomer thereof.

The invention also relates to the use of racetam and L-carnitine, or pharmaceutically acceptable salts thereof, in the manufacture of a medicament for the treatment and/or prevention of a mitochondrial disturbance in a mammal.

The invention also relates to the use of racetam and L-carnitine, or pharmaceutically acceptable salts thereof in the manufacture of a medicament for the treatment and/or prevention of mitochondrial disturbance related side effect induced by a drug in a mammal as a sixth major embodiment.

The invention also relates to the use according to the sixth major embodiment, wherein said mitochondrial disturbance is myopathy.

The invention also relates to the use according to the sixth major embodiment, wherein said mitochondrial disturbance is a neurodegenerative dysfunction and said neurodegenerative dysfunction can be selected from the group consisting of Alzheimer's disease and Parkinson's disease.

The invention further relates to a method of treating and/or preventing mitochondrial disturbances, said method comprising administering to a mammal a combination of racetam and L-carnitine as a seventh major embodiment.

The invention further relates to a method as described above, wherein said mitochondrial disturbance is myopathy.

The invention further relates to a method as described above, wherein said mitochondrial disturbance is a neurodegenerative dysfunction and said neurodegenerative dysfunction can be selected from the group consisting of Alzheimer's disease and Parkinson's disease.

The invention further relates to a method as described above, wherein said administration is a single dose or multiple doses to said mammal.

The invention further relates to a method of treating and/or preventing mitochondrial disturbance related side effect induced by a drug, said method comprising administering to a mammal a combination of racetam and carnitine.

The following examples are additional embodiments of the invention. However, it should be understood that such examples and embodiments are only provided for illustrative purposes, and different modifications or changes, in light of the provided embodiments, are familiar to practioners in the art and should be included in the spirit and scope of the invention.

### EXAMPLES

In the examples presented below, examples were realized with mitochondria isolated from liver of rat with the objective of demonstrating protective activity of the compounds L-carnitine, piracetam, and combinations thereof, in response to mitochondrial swelling provoked by statins and other substances.

Mitochondria were isolated from adult rat liver (MFR) using the technique of differential centrifugation, according to SCHNEIDER and HOGEBOOM (1951), after a fast of 12h. The liver, removed after death of the animal by cerebral concussion, was washed in a 250 mM saccharose solution containing buffer 10 mM HEPES pH 7.2 and 0.5 mM EGTA, and chopped with a scissors and homogenized in a

Potter-Elvehjem homogeneizer. The material was centrifuged at 2500xg for 10 minutes. The resulting supernatant was centrifuged for 10 minutes at 8000xg. The supernatant was discarded and the sediment resuspended in 250mM saccharose, 5mM HEPES pH 7.2, 0.3 mM EGTA, and centrifuged as the previous step. The mitochondrial fraction was ressuspensed in the same solution without EGTA, in a concentration of about 80mg of protein per milliliter of mitochondria suspension.

The experiments with isolated mitochondria were realized at 30°C in a standard reaction containing 125mM saccharose, 65mM potassium chloride, 10mM HEPES pH 7.2, and 2 mM phosphate. The substrate utilized was 5mM malate, pyruvate, glutamate, and α-ketoglutamate. Other reagents used are indictated in the figures.

Mitochondrial swelling was calculated based on the decrease in tubidity of a mitochondrial suspension measured at 520nm in a Hitachi spectrometer model U-3000 for a time period between 0 and 800 seconds.

This procedure of evaluation of mitochondria modification is based on fact that the mitochondria suspensions are turbid and scatter incident light. The scattered light is a function of the difference between the index of refraction of mitochondrial matrices, and accordingly, any process that decreases this difference will decrease the scattered light and increase the transmittance (see Nicholls, D.G., and Åkerman, K.E.O., Mitochondrial calcium transport. Biochim. Biophys. Acta 683, 57-88 (1982)). In this way, an increase in the volume of the mitochondrial matrix, associated with the entrance of permeable solutes, results in a consequent decrease of the scattered light. A spectrofotometric measurement of the absorbance reduction at 540nm (Ca²⁺-dependent NAD(P)⁺-induced alterations in membrane permeability of the mitochondria. Integration of Mitochondrial Function, Ed. J. Lemasters, Plenum, New York, pp. 535-542) is made in a spectrophotometer connected to a potentiometric recorder. The mitochondria (0.5mg protein/ml) were incubated in the reaction, and experiments were realized at a temperature of 30°C.

### EXAMPLE 1 - TEST in vitro of the MPT response to Calcium and Simvastatin.

A second protocol, different from above, was used for experiments in the Examples. Mitochondria from the livers of rat or mice were isolated by convential differential centrifugation (see description of method in Kaplan RS, Pedersen PL. Characterization of phosphate efflux pathways in rat liver mitochondrial. Biochem J 1983;212:279-88), using adult animals fasted for 12 hours. The livers were homogenized in 250mM saccharose, 1mM EGTA, and buffer 10mM Hepes (pH 7.2). The mitochrondrial suspension was washed twice, and the final precipitate was ressuspended in solution of 250 mM saccharose until a final concentration of 80-100 mg/ml of protein.

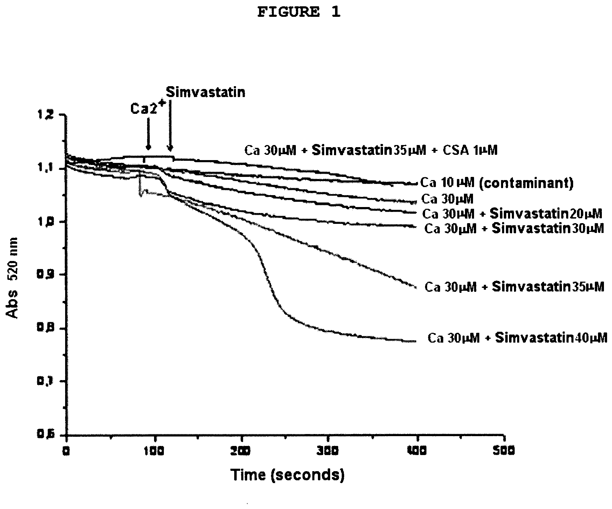

Figure 1 illustrates a dose response curve to calcium and simvastatin. The results show that simvastatin induces MPT. In particular, 40µM simvastatin plus 30µM Ca²⁺ induced the greatest increase in swelling of mitochondria. In the figure, Ca refers to calcium, simvastatina refers to simvastatine, tempo refers to time, and segundos refers to seconds.

### EXAMPLE 2 - Effect of the combination of L-carnitine and Piracetam on mitochondrial swelling induced by statins

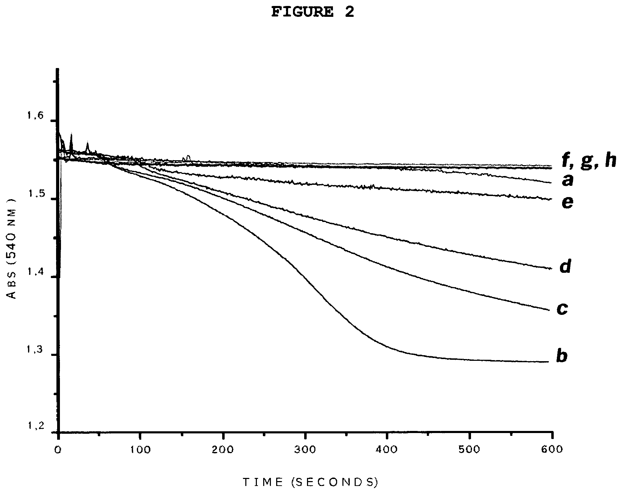

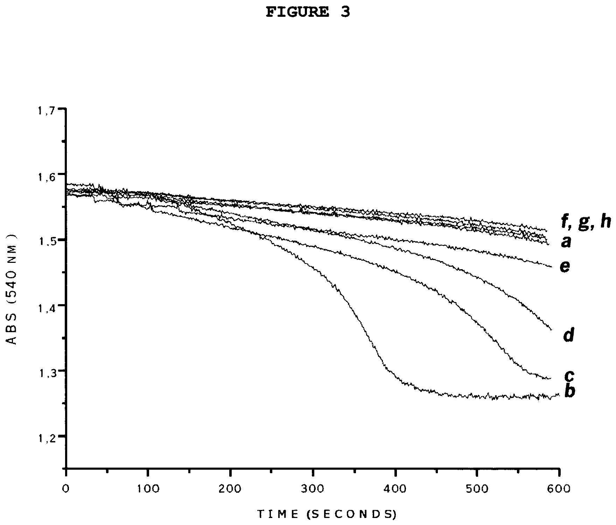

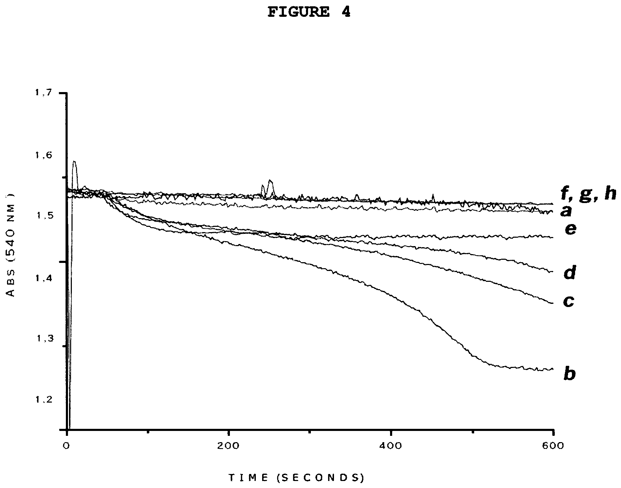

After determining the smallest dose of L-carnitine and the smallest dose of piracetam that presented some protective effect on mitochondrial swelling induced by statins (Simvastatin, Pravastatin and Lovastatin), the eficacy of the combination (L-carnitine + Piracetam) was evaluated at the same concentrations. Accordingly, it was determined that for Simvastatin and Pravastatin, a combination of 0.5µg/ml L-carnitine + 1.25 µg/ml Piracetam would be utilized (Figures 2 and 3 respectively); and for Lovastatin, a combination of 0.5µg/ml L-carnitine + 1.25µg/ml Piracetam would be utilized (Figure 4).

In Figure 2, MFR (0.5mg/ml) were incubated in a standard reaction containing Simvastatin (line b), Simvastatin + 0.5µg/ml L-carnitine (line c), Simvastatin + 1.25µg/ml Piracetam (line d), and Simvastatin + 0.5µg/ml L-carnitine + 1.25µg/ml Piracetam (line e), and compared to mitochondria without addition of Simvastatin (control, line a). Additions of L-carnitine, Piracetam and L-carnitine + Piracetam to controlled mitochondrias are illustrated in lines f, g and h, respectively. In the figure, tempo refers to time, and segundos refers to seconds.

Figure 3 shows the spectrophotometic profile of MFR (0.5mg/ml) incubated in a standard reaction containing Pravastatin (line b), Pravastatin + 0.5µg/ml L-carnitine (line c), Pravastatin + 1.25µg/ml Piracetam (line d), and Pravastatin + 0.5µg/ml L-carnitine + 1.25µg/ml Piracetam (line e), and compared to mitochondria without addition of Pravastatin (control, line a). Additions of L-carnitine, Piracetam, and L-carnitine + Piracetam to controlled mitochondria are illustrated in lines f, g and h, respectively. In the figure, tempo refers to time, and segundos refers to seconds.

Figure 4 presents the result of the combination of L-carnitine and Piracetam on mitochondria swelling induced by Lovastatin. MFR (0.5mg/ml) were incubated in a standard reaction containing Lovastatin (line b), Lovastatin + 0.5µg/ml L-carnitine (line c), Lovastatin + 1.25µg/ml Piracetam (line d), and Lovastatin + 0.5µg/ml L-carnitine + 1.25µg/ml Piracetam (line e), and compared to mitochondria without addition of Lovastatin (control, line a). Additions of L-carnitine, Piracetam, and L-carnitine + Piracetam to controlled mitochondria are illustrated in lines f, g and h, respectively. In the figure, tempo refers to time, and segundos refers to seconds.

Therefore, it is possible to use of low concentrations of piracetam and L-carnitine in the composition of the present invention, and with this, avoid the side effects of higher concentrations of these two drugs, as well as, treat or prevent the adverse effects induced by statins, or therapeutic agents associated with mitochondrial and/or metabolic disturbance or conditions related to oxidative stress, such as hypoxia, hypoglycemia, aging, and others.

### EXAMPLE 3 - Effect of Coenzyme Q10, alone or combined with L-carnitine and Piracetam (Piracar), on mitochondria swelling induced by Simvastatin

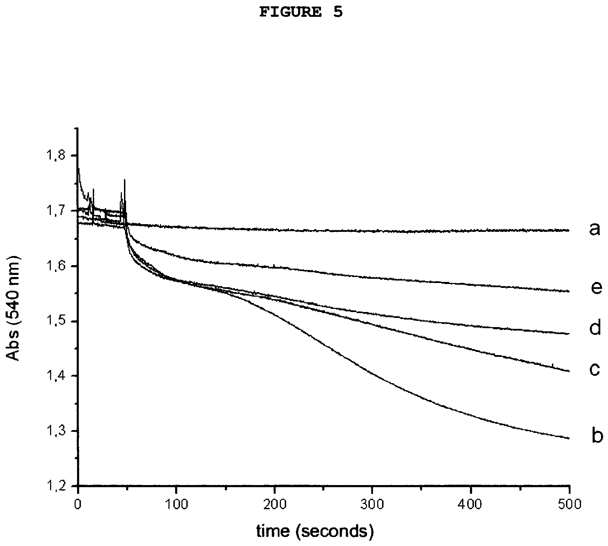

Figure 5 shows the spectrophotometic profile of MFR (0.5mg/ml) incubated in a standard reaction containing Simvastatin (line b), Simvastatin + Piracar 0.25/0.65 µg/ml (line c), Simvastatin + CoQ 5 µg/ml (line d), Simvastatin + CoQ 5 µg/ml + Piracar 0.25/0.65 µg/ml (line e); and compared to mitochondria without addition of Simvastatin (control, line a). These results again demonstrate the synergistic effect of the combination of L-carnitine and piracetam (PIRACAR), and the effect of the addition of coenzyme Q10 in the protection against mitochondria swelling. In the figure, tempo refers to time, and segundos refers to seconds.

### EXAMPLE 4 - COMPOSITION OF THE INVENTION IN A TABLET FORM

The composition of the invention can be in the form of a tablet or a coated tablet.

In the form of tablet, a mixture is initially made with the active ingredients and a vehicle and necessary additives, not only for obtainment of a formulation appropriated for compression, but also for obtainment of a medication with adequate shelf life. The components and the combination thereof, as well as production techniques, are known in the art of pharmacotechniques and pharmaceutical production.

In the following table (a), as an example, are formulations of the combination piracetam + carnitine according to the invention.

### (a) Formulation of the tablet or tablet core

| **Component** | **mg/cpo*** | **mg/cpo** |
|---|---|---|
| L-Carnitine | 250 | 500 |
| Piracetam | 250 | 350 |
| Lactose | 332 | 332 |
| Cellulose | 83 | 97.5 |
| Polyvinylpirrolidone | 25 | 25 |
| Collodial silicon dioxide | 20 | 5 |
| Croscarmellose sodium | 30 | 30 |
| FD&C Yellow no. 6 | - | 0.5 |
| Magnesium stearate | 10 | 10 |

| | | |
|---|---|---|
| * mg/cpo = mg/tablet | | |

The formulations in table (a) are able to constitute, also, the core of a coated tablet, where the formulation of the coating can be as illustrated in table (b).

### (b) Formulation for coating of coated tablet

| **Coating** | **mg/cpo** |
|---|---|
| Methacrylic acid copolymer | 100 |
| Triethyl citrate | 10 |
| Talcum | 40 |
| Water | 95 |
| Isopropilic alcohol | 705 |

### EXAMPLE 5 - COMPOSITION OF THE INVENTION IN A CAPSULE FORM

The composition of the invention can also be in the form of a capsule, which can be one of two types: a hard gelatinous capsule or a soft gelatinous capsule. The techniques for filling and manipulation of capsules are known in the state of the art. The composition of the invention can have the following formulation as shown for a hard gelatinous capsule.

| Component | Weight/capsule | Proportion % of the component per capsule |
|---|---|---|
| Piracetam | 350.00 mg | 35.00 % |
| L-Carnitine | 500.00 mg | 50.00 % |
| Microcristaline cellulose | 146.50 mg | 14.65 % |
| Magnesium stearate | 3.50 mg | 0.35 % |
| Hard gelatinous capsule | 1 cap. | 1 cap. |

### EXAMPLE 6 - COMPOSITION OF THE INVENTION IN THE FORM OF AN INJECTABLE SUSPENSION

The composition of the invention can be prepared in an injectable form according with techniques known in the state of the art. One of the major cares in this type of preparation relates to the guarantee of vehicle purity, that need to be absent of pyrogenic components and endotoxins. The composition of the invention can have the following formulation as shown for an injectable suspension.

| **Component** | **Weight/ampule** |
|---|---|
| Piracetam | 500.00 mg |
| L-Carnitine | 500.00 mg |
| Sodium acetate | 28 mg |
| Glacial acetic acid | 12.44 mg |
| Water for injection | q.s.p - 2.0 mL |

### EXAMPLE 7 - COMPOSITION OF THE INVENTION IN THE FORM OF AN ORAL SOLUTION

A preferred form of the composition of the invention is an oral solution, and it can be prepared by techniques known in the state of the art. A non-limiting example of an oral solution of the present invention is shown in the following table.

| **Component** | **Weight/ampule** | **Proportion % of component for pharmaceutical presentation** |
|---|---|---|
| Piracetam | 350.00 mg | 7.00% |
| L-Carnitine | 500.00 mg | 10.00% |
| Sodium cyclamate | 15.00 mg | 0.30% |
| Coloring | 0.02 mg | 0.0004% |
| Aroma | 0.85 mg | 0.017% |
| Methylparaben | 7.50 mg | 0.15% |
| Propylparaben | 0.50 mg | 0.01% |
| Purified water | q.s.p. 5.0 mL | q.s.p. 100.00 % |
| Hydrochloric acid | q.s. pH | q.s. pH |
| Sodium hydroxide | q.s. pH | q.s. pH |

The solution for oral administration as shown in the table above can be packed in an appropriate pharmaceutical presentation, such as, a vial, ampule, etc., wherein the preferred presentation is the vial.

All publications and patent applications mentioned in the description are indicated for easy reference for practioners of the relevant art.

The invention is explained by way of description, illustration, and examples, for the purpose of clearness and understanding, and it will be obvious that variations and modifications can be practiced within the scope of this description and the accompanying claims.

## Claims

1. Use of piracetam and L-carnitine, or pharmaceutically acceptable salts thereof, in the manufacture of a medicament for the treatment and/or prevention of myopathy induced by a statin, a pharmaceutically acceptable salt thereof or an isomer thereof, in a mammal.

2. Use according to claim 1, wherein said statin is selected from the group consisting of simvastatin, lovastatin, atorvastatin, pravastatin, fluvastatin, dalvastatin, cerivastatin, mevastatin, pitavastatin, rosuvastatin, a pharmaceutically acceptable salt thereof, or an isomer thereof.

## Patentansprüche

1. Verwendung von Piracetam und L-Carnitin, oder pharmazeutisch annehmbaren Salzen davon, in der Herstellung eines Arzneimittels für die Behandlung und/oder Vorbeugung von einer durch ein Statin, ein pharmazeutisch annehmbares Salz davon oder ein Isomer davon induzierten Myopathie in einem Säuger.

2. Verwendung nach Anspruch 1, wobei das Statin aus der Gruppe bestehend aus Simvastatin, Lovastatin, Atorvastatin, Pravastatin, Fluvastatin, Dalvastatin, Cerivastatin, Mevastatin, Pitavastatin, Rosuvastatin, einem pharmazeutisch annehmbaren Salz davon oder einem Isomer davon ausgewählt ist.

## Revendications

1. Utilisation de piracétam et de L-carnitine, ou des sels pharmaceutiquement acceptables de ceux-ci, dans la fabrication d'un médicament pour le traitement et/ou la prévention d'une myopathie induite par une statine, un sel pharmaceutiquement acceptable de celle-ci ou un isomère de celle-ci chez un mammifère.

2. Utilisation selon la revendication 1, dans laquelle ladite statine est choisie dans le groupe constitué de la simvastatine, la lovastatine, l'atorvastatine, la pravastatine, la fluvastatine, la dalvastatine, la cérivastatine, la mévastatine, la pitavastatine, la rosuvastatine, un sel pharmaceutiquement acceptable de celles-ci, ou un isomère de celles-ci.
